Europäisches Patentamt

European Patent Office

Office européen des brevets

(11) Publication number: **0 207 550 B1**

# EUROPEAN PATENT SPECIFICATION

(45) Date of publication of the patent specification:
**19.09.90**

(51) Int. Cl.⁵: **B01J 23/66, C07D 301/10**

(21) Application number: **86201019.6**

(22) Date of filing: **11.06.86**

(54) Process for the preparation of a silver-containing catalyst.

(30) Priority: **28.06.85 NL 8501864**

(43) Date of publication of application:
**07.01.87 Bulletin 87/2**

(45) Publication of the grant of the patent:
**19.09.90 Bulletin 90/38**

(84) Designated Contracting States:
**AT BE CH DE FR GB IT LI LU NL SE**

(56) References cited:
**EP-A- 0 001 371**
**EP-A- 0 058 419**
**GB-A- 1 560 480**
**US-A- 3 651 167**
**US-A- 4 207 210**

(73) Proprietor: **SHELL INTERNATIONALE RESEARCH MAATSCHAPPIJ B.V., Carel van Bylandtlaan 30, NL-2596 HR Den Haag(NL)**

(72) Inventor: **Boxhoorn, Gosse, Badhuisweg 3, NL-1031 CM Amsterdam(NL)**
Inventor: **Klazinga, Aan Hendrik, Badhuisweg 3, NL-1031 CM Amsterdam(NL)**
Inventor: **Velthuis, Otto Mente, Badhuisweg 3, NL-1031 CM Amsterdam(NL)**

(74) Representative: **Aalbers, Onno et al, P.O. Box 302, NL-2501 CH The Hague(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of a silver-containing catalyst suitable for the preparation of ethylene oxide, to a new catalyst and to the use of the catalyst for the preparation of ethylene oxide, and the ethylene oxide thus obtained.

It is generally known for a silver-containing catalyst to be employed in the preparation of ethylene oxide from ethylene. See for example British patent specification No. 1413251 and also the literature cited therein. In order to obtain improved silver catalysts, efforts have been directed for many years towards modifying the silver catalysts with the aid of promoters. For example, in the above-mentioned British patent specification No. 1413251 is described a process in which a silver compound is applied to a carrier, after which the applied silver compound is reduced to silver and in which additionally a promoter in the form of potassium oxide, rubidium oxide or cesium oxide or a mixture thereof is present on the carrier. In the course of time, all silver catalysts in service become less stable in the sense that their activity and selectivity decrease, and it becomes more economical to replace the used silver catalysts by fresh catalysts.

The applicant has found new silver catalysts with an improved stability.

The invention relates to a process for the preparation of a silver-containing catalyst suitable for the oxidation of ethylene to ethylene oxide, characterized in that a silver compound is applied to a carrier, after which the silver compound is reduced to metallic silver, and in which process the carrier has been prepared by mixing an aluminium compound with a salt of a metal of Group 1A of the Periodic System and by calcining the obtained mixture.

The invention also relates to a silver catalyst, suitable for the oxidation of ethylene to ethylene oxide comprising silver as an active component and an alkali enriched alumina as a carrier.

The US patent specification No. 4,379,134 discloses a process for the preparation of alpha-aluminium oxide, wherein a peptizing acid, water and fluoride anion are mixed with aluminium oxide, the resulting mixture being extruded to shaped particles which are calcined at a temperature of 400 °C to 700 °C for sufficient time to convert the aluminium oxide into gamma-aluminium oxide, after which these particles are calcined at a temperature of 1200 °C to 1700 °C for sufficient time to convert the gamma-aluminium oxide into alpha-aluminium oxide. The descriptive part of the specification also mentions that the fluoride acts as flux to enable sintering of aluminium oxide to take place at lower temperatures than the sintering temperature of pure aluminium oxide. HF is employed in the process according to all the examples of the US patent specification No. 4,379,134. The descriptive part mentions further that a number of metal fluorides are also suitable as flux, but there are no experiments to support this. Reference is also made to the use of silicon dioxide and alkali metal oxides as fluxes, but this use is immediately rejected, since these fluxes could introduce undesirable impurities into aluminium oxide.

It is therefore surprising that the applicant has found that metals of Group 1A of the Periodic System, especially potassium, rubidium or cesium, in the form of their salts can be mixed in the carrier, thereby leading to improved silver catalysts with improved stability.

The aluminium compounds can be a variety of modifications of aluminium oxide, which when calcined at between 1200 °C and 1700 °C produce alpha-aluminium oxide, such as gamma-aluminium oxide. Another possibility is to choose a hydrated aluminium oxide, such as boehmite, which via gamma-aluminium oxide produces alpha-aluminium oxide.

The salts of the metals of Group 1A of the Periodic System may be, for example, fluorides, nitrates, chlorides or sulphates. The metals are lithium, sodium, potassium, rubidium or cesium. Preferably, potassium, rubidium or cesium salts are used. Cesium fluoride or cesium nitrate are particularly suitable. Lithium fluoride and lithium nitrate have also been found to give good results.

The quantity of salt of the alkali metal that is mixed with the aluminium compound is chosen such that the atom ratio of the metal of Group 1A/Al is between 0.0001 and 0.1, preferably between 0.001 and 0.01.

For the preparation of the enriched carrier, preferably an aluminium compound is mixed with water and a salt of a metal of Group 1A of the Periodic System, the mixture thus obtained being extruded to shaped particles which are subsequently calcined. The calcination can take place in one or more steps, depending on the choice of starting material. In general, sufficient water is added to make the mixture extrudable. The extrudable paste obtained is then extruded in an extruder to form shaped pieces. These shaped pieces are heated, during which water still present is evaporated. The solid pieces are calcined. In order to prepare the alpha-aluminium oxide modification, calcination up to a temperature of between 1200 °C and 1700 °C is necessary. Suitable starting materials are powders of gamma-aluminium oxide, alpha-aluminium oxide monohydrate, alpha-aluminium oxide trihydrate and beta-aluminium oxide monohydrate, which are sintered during the calcination, with fusion of the powder particles taking place. The heating and calcination also changes the crystal structure: the cubic structure of gamma- aluminium oxide changes into the hexagonal structure of alpha- aluminium oxide.

The effective catalyst surface area can vary from between 0.2 and 5 m²/g. It has also been found that for the alpha-aluminium oxide, the alkali metal (cesium) is present at the surface at a concentration of about six times higher than is to be expected on the basis of the weighed-out quantity of alkali metal.

In order to prepare a catalyst, the alkali enriched carrier is impregnated with a solution of a silver compound, sufficient to apply, as wished, 1 to 25 weight per cent of silver, calculated on the weight of the total catalyst, to the carrier. The impregnated carrier is separated from the solution and the precipitated silver compound is reduced to silver.

Preferably a promoter is added, for example one or more of the alkali metals: potassium, rubidium or cesium. The promoters can be applied to the carrier before, during or after the impregnation with silver compound. The promoter can also be applied to the carrier after the silver compound has been reduced to silver.

In general, the carrier is mixed with an aqueous solution of a silver salt or silver complex, so that the carrier is impregnated with this solution, after which the carrier is separated from the solution and subsequently dried. The impregnated carrier is then heated to a temperature of between 100 °C and 400 °C for a period necessary for the silver salt (or complex) to decompose and form a finely distributed layer of metallic silver which adheres to the surfaces.

Heating of the impregnated carrier to 500 °C may optionally be applied during, for example, one hour. Thereafter an alkali metal may be applied on the carrier (post-doping with cesium). A reducing or inert gas can be passed over the carrier during the heating.

Various methods are known for adding the silver. The carrier can be impregnated with an aqueous solution of silver nitrate, then dried, after which the silver nitrate is reduced with hydrogen or hydrazine. The carrier can also be impregnated with an ammoniacal solution of silver oxalate or silver carbonate, the deposition of silver metal being effected by thermally decomposing the salt. Special solutions of a silver salt with certain solubilizing and reducing agents, such as combinations of vicinal alkanolamines, alkyldiamines and ammonia also serve the purpose.

The quantity of added promoter is generally between 20 and 1000 parts by weight of an alkali metal, such as potassium, rubidium or cesium (as metal) per million parts by weight of total catalyst. 50 to 300 parts by weight of alkali metal is particularly suitable. Suitable compounds to serve as starting material for promoters are, for example, nitrates, oxalates, carboxylic acid salts or hydroxides. The most preferred promoter is cesium, the cesium being preferably employed as cesium hydroxide or cesium nitrate.

Some excellent methods are known for adding the alkali metals in which these metals can be applied at the same time as the silver. Suitable alkali metal salts are generally salts which are soluble in the silver-depositing liquid phase. Besides the above-mentioned salts, it is also worth mentioning nitrates, chlorides, iodides, bromides, bicarbonates, acetates, tartrates, lactates and isopropoxides. The use of alkali metal salts which react with the silver present in the solution and thus cause silver salts to be prematurely precipitated from an impregnating solution should, however, be avoided. For example, potassium chloride should not be used for impregnating techniques in which an aqueous silver nitrate solution is used, but potassium nitrate can be used instead. Potassium chloride can be suitably used in a process in which an aqueous solution of silver amine complexes, from which no silver chloride will precipitate, is used.

In addition, the amount of alkali metal deposited on the carrier can be adjusted within certain limits by washing out a part of the alkali metal with, preferably, anhydrous methanol or ethanol. This method is employed subsequently if the concentration of the applied alkali metal is found to be too high. The temperatures, contact times and the drying with gases can be adjusted. Care should be taken to ensure that no traces of alcohol remain in the carrier.

A preferably employed process consists of the carrier being impregnated with an aqueous solution containing both alkali metal salts and silver salts, the impregnating solution being composed of a silver salt of a carboxylic acid, an organic amine, a salt of potassium, rubidium or cesium and an aqueous solvent. For example, a potassium-containing silver oxalate solution can be prepared in two ways. Silver oxide can be reacted with a mixture of ethylene diamine and oxalic acid, giving a solution containing a silver oxalate ethylene diamine complex, to which a certain amount of potassium and possibly other amines such as ethanolamine is added. Silver oxalate can also be precipitated from a solution of potassium oxalate and silver nitrate, the silver oxalate thus obtained then being repeatedly washed in order to remove the attached potassium salts until the desired potassium content is obtained. The potassium-containing silver oxalate is then solubilized with ammonia and/or amine. Solutions containing rubidium and cesium can also be prepared in this way. The thus impregnated carriers are then heated to a temperature of between 100 °C and 400 °C, preferably between 125 °C and 325 °C.

It should be noted that, irrespective of the nature of the silver in the solution before the precipitation onto the carrier, reference is always made to reduction to metallic silver, whereas it could also be referred to as decomposition on heating. It is preferred to think in terms of reduction, since positively charged Ag ions are converted into metallic Ag. The reduction times can be simply adapted to the starting materials employed.

As mentioned above, a promoter is preferably added to the silver. Cesium is the most preferred promoter in view of the fact that its selectivity for ethylene oxide has been found to be the highest in comparison with the use of potassium or rubidium as promoter.

The silver catalysts prepared by the process according to the present invention appear to be particularly stable catalysts for the direct catalytic oxidation of ethylene to ethylene oxide with the aid of molecular oxygen. The conditions for carrying out the oxidation reaction in the presence of the silver catalysts according to the invention are fairly similar to those already described in the literature. This applies to, for example, suitable temperatures, pressures, residence times, diluents such as nitrogen, carbon dioxide, steam, argon, methane or other saturated hydrocarbons, the presence or absence of moderating agents to control the catalytic action, for example 1,2-dichloroethane, vinyl chloride or chlorinated polyphenyl compounds, the desirability of employing either recirculating treatments or successive conversions in different reactors to enhance the yield of ethylene oxide, as well as any other special conditions which may be chosen for processes for the preparation of ethylene oxide. Usually, the pressures employed vary from about atmospheric pressure to about 35 bar. Higher pressures are, however, by no means excluded. The molecular oxygen employed as reactant can be obtained from conventional sources. The oxygen feed can consist of substantially pure oxygen, of a concentrated oxygen stream consisting of a large amount of oxygen with smaller amounts of one or more diluents, such as nitrogen, argon, etc., or of another oxygen-containing stream, such as air.

In a preferably employed application of the silver catalysts according to the present invention, ethylene oxide is prepared by contacting an oxygen-containing gas that has been separated from air and that contains not less than 95% oxygen with ethylene in the presence of the catalysts in question at a temperature within the range of 210 °C to 285 °C and preferably between 225 °C and 270 °C. Gas hourly space velocities may range from 2800 to 8000 h$^{-1}$.

In the reaction of ethylene with oxygen to ethylene oxide, the ethylene is present in at least a double molecular quantity, but the quantity of ethylene employed is generally much higher. The conversion is therefore calculated according to the quantity of converted oxygen in the reaction and we therefore speak of oxygen conversion. This oxygen conversion is dependent on the temperature of the reaction and is a measure of the activity of the catalyst. The values $T_{30}$, $T_{40}$ and $T_{50}$ refer to the temperatures at 30 mol%, 40 mol% and 50 mol% conversion respectively of the oxygen in the reactor. The temperatures are generally higher for a higher conversion and are highly dependent on the catalyst employed and the reaction conditions. In addition to these T-values, one also comes across selectivity values, which indicate the molar percentage of ethylene oxide in the reaction mixture obtained. The selectivity is indicated as $S_{30}$, $S_{40}$ or $S_{50}$, which refers to the selectivity at 30%, 40% or 50% oxygen conversion respectively.

The concept "stability of a catalyst" cannot be expressed directly. Stability measurements require trials of long duration. For measuring the stability, the applicant has a test which is carried out under extreme conditions with space velocities in the order of 30,000 litre.(litre catalyst)$^{-1}$.h$^{-1}$, where litres of throughput gas are understood to be litres STP (Standard temperature and pressure). This space velocity is many times higher than the space velocity in commercial processes. The test is carried out for at least 1 month. The above-mentioned T- and S-values are measured during the entire period of the test. After the test has been broken off, the total quantity of ethylene oxide produced per ml catalyst is determined. The difference in selectivity and activity is calculated for a catalyst which would have produced 1000 grams ethylene oxide per ml catalyst. A new catalyst is considered to be more stable than a known catalyst if the differences in the T- and S-values of the new catalyst are less than those of the standard catalyst which is present during each test. The stability tests are generally carried out at 35% oxygen conversion.

Example 1

1 g cesium fluoride dissolved in 160 ml water was mixed with 137.5 g Kaiser aluminium oxide (26102) ($Al_2O_3.H_2O$) by adding the cesium fluoride solution to the aluminium oxide, and the mixture was kneaded for 10 minutes in a masticator. The paste obtained was left to stand for three hours and then extruded. The resulting shaped pieces were dried for 3 hours at 120 °C and subsequently calcined at progressively higher temperatures. Calcination was started with the temperature rising at a rate of 200 °C/h to 700 °C. Calcination was then continued for 1 hour at 700 °C, after which the temperature was raised in 2 hours to 1600 °C. Finally, calcination was continued for 1 hour at 1600 °C. The pore volume of the shaped aluminium oxide pieces was 0.54 ml.g$^{-1}$ and the average pore diameter was 2.0 µm. The weighed-out cesium/aluminium atom ratio was 0.003, while the cesium/aluminium atom ratio at the pore surface was 0.019. The resulting shaped pieces were impregnated with an aqueous solution of silver oxalate, to which cesium hydroxide was added. The impregnation was carried out for 10 minutes under vacuum, after which the shaped pieces were separated from the solution and placed in a hot air stream at a temperature of 250-270 °C for 10 minutes in order to convert the silver salt to silver. The aqueous solution of silver oxalate was a 28 wt% Ag-containing aqueous solution in which the silver oxalate was complexed with ethylene diamine and to which solution cesium hydroxide was added. After the hot air treatment the thus impregnated shaped pieces contained 19.1 wt% Ag (calculated on total catalysts) and 100 parts by weight of cesium per million parts by weight of total catalyst.

The catalyst obtained was then tested. A cylindrical steel reactor with a length of 15 cm and a cross-section of 3 mm was filled entirely with catalyst particles of about 0.3 mm in size. The reactor was placed in a bath in which silicon/aluminium particles were present in a fluidized state. A gas mixture with the following composition was passed through the reactor: 30 mol% ethylene, 8.5 mol% oxygen, 7 mol% carbon dioxide and 54.5 mol% nitrogen and 7 parts per million parts of gas, of vinyl chloride as moderator. The space velocity was 30,000 1.1$^{-1}$.h$^{-1}$. The pressure was 15 bar and the temperature was dependent on the set oxygen conversion. The measuring equipment was connected to the reactor and to a computer such that the conversion and the temperature could be accurately controlled. The concentrations of the reaction components were determined with the aid of gas chromatography and mass spectrometry. The stability test was carried out at an oxygen conversion of 35%.

The reaction temperature at 35% oxygen conversion was determined during the entire duration of the test. The selectivity in respect of ethylene oxide was also determined. After at least 30 days the test was broken off and the total quantity of ethylene oxide produced per ml catalyst was determined. From the measured reaction temperatures the temperature rise in °C was calculated for the moment at which 1000 g ethylene oxide per ml catalyst would have been produced

$$(T^{1000}_{35}).$$

From the measured selectivities, the selectivity decrease in mol% was calculated for the moment at which 1000 g ethylene oxide per ml catalyst would have been produced

$$(S^{1000}_{35}).$$

The same measurements and calculations were carried out for a standard catalyst in the test.

The table shows the

$$S^{1000}_{35} \text{ and } T^{1000}_{35}$$

relative to those of the standard catalyst.

Example 2

A catalyst was prepared and tested in the same way as in Example 1, 17.3 wt% Ag and 160 parts by weight cesium per million parts by weight of total catalyst being used. 5.5 ppm vinyl chloride was used as moderator in this test.

| Example | Catalyst wt.% Ag | ppm Cs | Duration Days | Total wt. of eth. oxide produced per ml cat. | $\Delta S^{1000}_{35}$ (%m) | $\Delta T^{1000}_{35}$ (°C) |
|---|---|---|---|---|---|---|
| 1 | 19.1 | 100 | 30 | 900 g | 1.8 | 12 |
|  | S839 |  | 36 | 1105 g | 2.3 | 11 |
| 2 | 17.3 | 160 | 35 | 960 g | 1.4 | 9.5 |
|  | S839 |  | 35 | 950 g | 1.9 | 19 |

S839 = a commercial catalyst comprising silver on a non alkali enriched carrier

## Claims

1. A process for the preparation of a silver-containing catalyst suitable for the oxidation of ethylene to ethylene oxide, characterized in that a silver compound is applied to a carrier, after which the silver compound is reduced to metallic silver, and in which process the carrier has been prepared by mixing an aluminium compound with a salt of a metal of Group 1A of the Periodic System and by calcining the obtained mixture.

2. A process according to claim 1, characterized in that the aluminium compound is an aluminium oxide or a hydrate of aluminium oxide.

3. A process according to claim 1 or 2, characterized in that the aluminium compound is mixed with a fluoride, a nitrate, a chloride or a sulphate of a metal of Group 1A of the Periodic System.

4. A process according to one or more of claims 1-3, characterized in that the aluminium compound is mixed with cesium fluoride or cesium nitrate.

5. A process according to one or more of claims 1-4, characterized in that the aluminium compound is mixed with a quantity of salt such that the atom ratio of metal of Group 1A/Al is between 0.0001 and 0.1.

6. A process according to claim 5, characterized in that the atom ratio is between 0.001 and 0.01.

7. A process according to one or more of claims 1-6, characterized in that the aluminium compound is boehmite or gamma-aluminium oxide.

8. A process according to one or more of claims 1-7, characterized in that the aluminium compound is mixed with water and a salt of a metal of Group 1A of the Periodic System, the resulting mixture being extruded to shaped carrier particles which are then calcined.

9. A process according to one or more of claims 1-8, characterized in that the carrier is impregnated with a solution of a silver compound sufficient to apply 1 to 25 weight per cent of silver, calculated on the weight of the total catalyst on the carrier, the impregnated carrier being separated from the solution and the precipitated silver compound being reduced to silver.

10. A process according to one or more of claims 1-9, characterized in that a promoter is applied to the carrier.

11. A process according to claim 9 or 10, characterized in that besides the silver compound applied to the carrier, a sufficient quantity of one or more compounds of the alkali metals potassium, rubidium or cesium is applied, either simultaneously or not, to the carrier to deposit between 20 and 1000 parts by weight of alkali metal (measured as metal) per million parts by weight of total catalyst.

12. A catalyst comprising
a) from 1 to 25 per cent by weight of silver, calculated on the weight of the total catalyst, on the surface of the carrier and
b) alkali enriched alpha-alumina as a carrier.

13. A catalyst according to claim 12, comprising also a promoter on the surface of the carrier.

14. A catalyst according to claim 13, comprising between 20 and 1000 parts by weight of potassium, rubidium or cesium (measured as the metal) per million by weight of total catalyst.

15. A process for the preparation of ethylene oxide by oxidation of ethylene in the presence of a silver-containing catalyst, characterized in that the process is carried out in the presence of a silver-containing catalyst prepared with the aid of the process according to one or more of claims 1-11 or one or more of claims 12-14.

## Patentansprüche

1. Verfahren zur Herstellung eines zur Oxidation von Ethylen zu Ethylenoxid geeigneten Silber enthaltenden Katalysators, dadurch gekennzeichnet, daß eine Silberverbindung auf einen Träger aufgebracht wird, wonach die Silberverbindung zu metallischem Silber reduziert wird, und in welchem Verfahren der Träger durch Vermischen einer Aluminiumverbindung mit einem Salz eines Metalles der Gruppe 1A des Periodensystems und durch Kalzinieren des erhaltenen Gemisches hergestellt worden ist.

2. Verfahren nach Anspruch 1, dadurch gekennzeichnet, daß die Aluminiumverbindung ein Aluminiumoxid oder ein Aluminiumoxidhydrat ist.

3. Verfahren nach Anspruch 1 oder 2, dadurch gekennzeichnet, daß die Aluminiumverbindung mit einem Fluorid, einem Nitrat, einem Chlorid oder einem Sulfat eines Metalles 1A des Periodensystems vermischt wird.

4. Verfahren nach einem oder mehreren der Ansprüche 1 bis 3, dadurch gekennzeichnet, daß die Aluminiumverbindung mit Cäsiumfluorid oder Cäsiumnitrat vermischt wird.

5. Verfahren nach einem oder mehreren der Ansprüche 1 bis 4, dadurch gekennzeichnet, daß die Aluminiumverbindung mit einer solchen Menge an Salz vermischt wird, daß das Atomverhältnis vom Metall der Gruppe 1A/Al von 0,0001 bis 0,1 beträgt.

6. Verfahren nach Anspruch 5, dadurch gekennzeichnet, daß das Atomverhältnis von 0,001 bis 0,01 beträgt.

7. Verfahren nach einem oder mehreren der Ansprüche 1 bis 6, dadurch gekennzeichnet, daß die Aluminiumverbindung Boehmit oder gamma-Aluminiumoxid ist.

8. Verfahren nach einem oder mehreren der Ansprüche 1 bis 7, dadurch gekennzeichnet, daß die Aluminiumverbindung mit Wasser und einem Salz eines Metalles der Gruppe 1A des Periodensystems vermischt wird und das erhaltene Gemisch zu geformten Trägerteilchen extrudiert wird, die dann kalziniert werden.

9. Verfahren nach einem oder mehreren der Ansprüche 1 bis 8, dadurch gekennzeichnet, daß der Träger mit einer Lösung einer Silberverbindung imprägniert wird, die zum Aufbringen von 1 bis 25 Gew.-% Silber, berechnet auf das Gewicht des Gesamtkatalysators, auf den Träger ausreicht, der imprägnierte Träger von der Lösung abgetrennt wird, und die ausgefällte Silberverbindung zu Silber reduziert wird.

10. Verfahren nach einem oder mehreren der Ansprüche 1 bis 9, dadurch gekennzeichnet, daß ein Promoter auf den Träger aufgebracht wird.

11. Verfahren nach Anspruch 9 oder 10, dadurch gekennzeichnet, daß zusätzlich zu der auf den Träger aufgebrachten Silberverbindung eine ausreichende Menge einer oder mehrerer Verbindungen der Alkalimetalle Kalium, Rubidium oder Cäsium, entweder gleichzeitig oder unabhängig auf den Träger aufgebracht wird, um 20 bis 1000 Gewichtsteile Alkalimetall (gemessen als Metall) je Million Gewichtsteile Gesamtkatalysator abzulagern.

12. Ein Katalysator, umfassend
a) 1 bis 25 Gew.-% Silber, berechnet auf das Gewicht des Gesamtkatalysators, auf der Oberfläche des Trägers, und
b) Alkali-angereichertes alpha-Aluminiumoxid als Träger.

13. Katalysator nach Anspruch 12, der zusätzlich auch einen Promotor an der Trägeroberfläche umfaßt.

14. Katalysator nach Anspruch 13, umfassend 20 bis 1000 Gewichtsteile Kalium, Rubidium oder Cäsium (gemessen als Metall) je Million Gewichtsteile Gesamtkatalysator.

15. Verfahren zur Herstellung von Ethylenoxid durch Oxidation von Ethylen in Gegenwart eines Silber enthaltenden Katalysators, dadurch gekennzeichnet, daß das Verfahren in Anwesenheit eines Silber enthaltenden Katalysators, der mit Hilfe des Verfahrens nach einem oder mehreren der Ansprüche 1 bis 11 hergestellt worden ist, oder nach einem oder mehreren der Ansprüche 12 bis 14 ausgeführt wird.

**Revendications**

1. Un procédé de préparation d'un catalyseur contenant de l'argent utilisable pour l'oxydation d'éthylène en oxyde d'éthylène, caractérisé en ce qu'un composé de l'argent est déposé sur un support, après quoi le composé de l'argent est réduit à l'état d'argent métallique, le support ayant été préparé en mélangeant un composé de l'aluminium avec un sel d'un métal du groupe 1A du système périodique et en calcinant le mélange obtenu.

2. Un procédé selon la revendication 1, caractérisé en ce que le composé de l'aluminium est un oxyde d'aluminium ou un hydrate d'oxyde d'aluminium.

3. Un procédé selon la revendication 1 ou 2, caractérisé en ce que le composé de l'aluminium est mélangé avec un fluorure, un nitrate, un chlorure ou un sulfate d'un métal du groupe 1A du système périodique.

4. Un procédé selon une ou plusieurs des revendications 1-3, caractérisé en ce que le composé de l'aluminium est mélangé avec du fluorure de césium ou du nitrate de césium.

5. Un procédé selon une ou plusieurs des revendications 1-4, caractérisé en ce que le composé de l'aluminium est mélangé avec une quantité de sel telle que le rapport atomique du métal du groupe 1A à l'aluminium soit compris entre 0,0001 et 0,1.

6. Un procédé selon la revendication 5, caractérisé en ce que le rapport atomique est compris entre 0,001 et 0,01.

7. Un procédé selon une ou plusieurs des revendications 1-6, caractérisé ence que le composé de l'aluminium est de la boehmite ou de l'oxyde d'aluminium gamma.

8. Un procédé selon une ou plusieurs des revendications 1-7, caractérisé en ce que le composé de l'aluminium est mélangé avec de l'eau et un sel d'un métal du groupe 1A du système périodique, le mélange résultant étant extrudé en particules de support de forme déterminée qui sont ensuite calcinées.

9. Un procédé selon une ou plusieurs des revendications 1-8, caractérisé en ce que le support est imprégné d'une solution d'un composé de l'argent d'une manière suffisante pour appliquer sur le support de 1 à 25 % en poids d'argent, par rapport au poids du catalyseur total, le support imprégné étant séparé de la solution et le composé de l'argent précipité étant réduit en argent.

10. Un procédé selon une ou plusieurs des revendications 1-9, caractérisé en ce qu'un promoteur est appliqué au support.

11. Un procédé selon la revendication 9 ou 10, caractérisé en ce qu'en plus du composé de l'argent appliqué au support, on applique au support, simultanément ou pas, une quantité suffisante d'un ou plusieurs composés des métaux alcalins potassium, rubidium ou césium pour déposer entre 20 et 1000 parties en poids de métal alcalin (en mesurant en métal) par million de parties en poids de catalyseur total.

12. Un catalyseur comprenant
a) de 1 à 25 % en poids d'argent, par rapport au poids du catalyseur total, sur la surface du support et
b) de l'alpha-alumine enrichie en métal alcalin comme support.

13. Un catalyseur selon la revendication 12, comprenant aussi un promoteur sur la surface du support.

14. Un catalyseur selon la revendication 13, comprenant entre 20 et 1000 parties en poids de potassium, de rubidium ou de césium (en mesurant en métal) par million de parties en poids de catalyseur total.

15. Un procédé de préparation d'oxyde d'éthylène par oxydation d'éthylène en présence d'un catalyseur contenant de l'argent, caractérisé en ce que le procédé est mis en oeuvre en présence d'un catalyseur contenant de l'argent préparé à l'aide d'un procédé selon une ou plusieurs des revendications 1-11 ou une ou plusieurs des revendications 12-14.